# EUROPEAN PATENT SPECIFICATION

(11) **EP 3 948 264 B1**
(45) Date of publication and mention of the grant of the patent: **06.05.2026**
(21) Application number: 20721338.0
(22) Date of filing: 28.03.2020
(51) Int. Cl.: G01N 31/22, G01N 21/78, G01N 33/18, G01N 21/77

(54) **NON-BLEACHING COLORIMETRIC AND FLUOROMETRIC ANALYTE DETECTION BY DEGRADATION OF SOLIDS**
NICHTBLEICHENDE KOLORIMETRISCHE UND FLUOROMETRISCHE ANALYTDETEKTION DURCH ABBAU VON FESTSTOFFEN
DÉTECTION D'ANALYTE DE COLORIMÉTRIE ET DE FLUORIMÉTRIE SANS BLANCHIMENT PAR DÉGRADATION DES SOLIDES

(30) Priority: 28.03.2019 US 201962825472 P
(43) Date of publication of application: 09.02.2022
(73) Proprietor: Hach Company, Loveland, CO 80538-8842 (US)
(72) Inventor: MIERITZ, Daniel, Gustav, Fort Collins, CO 80525 (US); GREENAWALT, Angella, Nicholle, Fort Collins, CO 80525 (US)
(74) Representative: Sandri, Sandro
(86) International application number: PCT/US2020/025588
(87) International publication number: WO 2020/198725

(56) References cited:
- YUEXIN GUO ET AL: "Tuning the Luminescence of Metal-Organic Frameworks for Detection of Energetic Heterocyclic Compounds", JOURNAL OF THE AMERICAN CHEMICAL SOCIETY, vol. 136, no. 44, 17 October 2014 (2014-10-17), pages 15485 - 15488, XP055583760, DOI: 10.1021/ja508962m
- HINTERHOLZINGER FLORIAN M ET AL: "Highly sensitive and selective fluoride detection in water through fluorophore release from a metal-organic framework", SCIENTIFIC REPORTS, vol. 3, 2562, 6 September 2013 (2013-09-06), pages 1 - 7, XP055720571, DOI: 10.1038/srep02562
- ZHAO XUDONG ET AL: "Fluorescent molecule incorporated metal-organic framework for fluoride sensing in aqueous solution", APPLIED SURFACE SCIENCE, ELSEVIER, NL, vol. 402, 11 January 2017 (2017-01-11), pages 129 - 135, XP029915916, ISSN: 0169-4332, DOI: 10.1016/J.APSUSC.2017.01.075
- LIN XIAOMEI ET AL: "Fast, Sensitive, and Selective Ion-Triggered Disassembly and Release Based on Tris(bipyridine)ruthenium(II)-Functionalized Metal-Organic Frameworks", ANALYTICAL CHEMISTRY, ACS, US, vol. 87, no. 9, 24 April 2015 (2015-04-24), pages 4864 - 4870, XP055720450, ISSN: 0003-2700, DOI: 10.1021/acs.analchem.5b00391

## Description

### BACKGROUND

The measurement of analytes (e.g., fluoride, borate, carbonate, mercury) in drinking water is an important task for water treatment facilities. With respect to fluoride, most municipal water facilities introduce a controlled amount of fluoride into drinking water. One benefit to this introduction of fluoride is that when the fluoride is ingested it slows the rate of tooth enamel demineralization and increases the rate of remineralization. This process reduces the incidence of tooth cavities in the population served by fluoridated water. However, high concentrations of fluoride can be detrimental. For example, if the fluoride concentration is too high, dental fluorosis may occur. Additionally, the facility is wasting resources by the addition of too much fluoride. On the other hand, if the fluoride concentration is too low, the prevention of tooth cavities suffers. Therefore, it is desired to closely monitor the level of fluoride in drinking water to achieve a desired concentration of fluoride, and to ensure compliance with regulations. Similarly, the concentrations of other analytes within drinking water are closely monitored in order to ensure compliance within a predetermined range or to ensure that the analyte is not present at all within the drinking water.

There are a number of methods to measure fluoride in drinking water. These include the SPADNS and ion selective electrode techniques. SPADNS requires the preparation of a blank sample vial, and because the chemistry involves the bleaching of a dye, differing styles of measurement may lead to inaccurate results. The ion selective technique requires the addition of an ionic strength adjustment buffer, and the equilibrium time for low levels of fluoride that are not within a linear range may be sensitive to sample movement and temperature leading to inaccurate results. Similar techniques are conventional for measuring other analytes.

Article HINTERHOLZINGER FLORIAN M ET AL; "Highly sensitive and selective fluoride detection in water through fluorophore release from a metal-organic framework", SCIENTIFIC REPORTS, vol. 3, 2562, 6 September 2013, pages 1-7, DOI: 10.1038/srep02562, describes the use of a porous crystalline framework for fluoride ion sensing, where the porous crystalline framework serves as a host for a fluorescent reporter molecule, whereby the detection is based on the decomposition of the host scaffold which induces the release of the fluorescent dye molecule.

Article ZHAO XUDONG ET AL: "Fluorescent molecule incorporated metal-organic framework for fluoride sensing in aqueous solution", APPLIED SURFACE SCIENCE, ELSEVIER, NL, vol. 402, 11 January 2017, pages 129-135, ISSN: 0169-4332, DOI: 10.1016/J.APSUSC.2017.01.075 describes a method according to which a fluorescent molecule was incorporated in a zirconium-based MOF.

Article GUO YUEXIN ET AL: "Tuning the Luminescence of Metal-Organic Frameworks for Detection of Energetic Heterocyclic Compounds", JOURNAL OF THE AMERICAN CHEMICAL SOCIETY, vol. 136, 17 October 2014, pages 15485-15488, DOI: 10.1021/ ja508962m describes a detection method for explosives using a Mg-, Ni- or Co-MOF which is not impregnated with a photoreactive species, as the detection is based on reassembling of the released linkers TABD-COOH to form emissive aggregates due to aggregation-induced emission.

### BRIEF SUMMARY

The present invention provides a method for measuring an ion component of an aqueous sample using a photoreactive species, comprising the steps disclosed at claim 1. The dependent claims outline advantageous ways of carrying out the method.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates an example of computer circuitry.
FIG. 2 illustrates a flow diagram of an example analyte detection and measuring system.
FIG. 3 illustrates a synthesis scheme of an example of analyte detection.
FIG. 4 illustrates an example raw absorbance curve of a CO₂ test of Al-NH₂-TPA MOF.
FIG. 5 illustrates the MOF dose response curve for the same test illustrated in FIG. 4, specifically a CO₂ test of Al-NH₂-TPA MOF.
FIG. 6 illustrates the delta absorbance of the test illustrated in FIG. 4 and FIG. 5, specifically a CO₂ test of Al-NH₂-TPA MOF.
FIG. 7 illustrates an example fluorescent response curve of NH₂-UiO-66 to CO₂.

### DETAILED DESCRIPTION

Colorimetry, Henry's law, thermal conductivity and electrochemical sensors may be utilized for analysis of carbon dioxide dissolved in aqueous solutions. Electrochemical sensors may employ a carbon dioxide permeable membrane, that selectively allows carbon dioxide to enter the sensor. In the sensor, a constant voltage may be applied between anode and cathode, and the observed current is proportional to the partial pressure of carbon dioxide. In aqueous solutions, carbon dioxide may exist as an equilibrium between dissolved carbon dioxide, carbonic acid, carbonate anions and bicarbonate anions, whose relative concentrations may be calculated as a function of pH and temperature. Colorimetric methods may rely on the color change of an acid-base indicator molecule such as thymol blue in basic solution, in which carbon dioxide dissolves and forms carbonic acid that dissociates to decrease the pH, resulting in a color change. Henry's law describes the equilibrium between the concentration of a dissolved species and the partial pressure of that species in the gas phase by employing a characteristic Henry's constant, at a certain temperature. This thermodynamic relationship may be utilized by measuring the differential temperature and pressure of the gas above a given liquid solution with a known volume of headspace, from which the amount of dissolved carbon dioxide may be calculated. In another method that utilizes thermal conductivity, the dissolved carbon dioxide may be liberated into the headspace by acidifying the sample, and the thermal conductivity of the gas may be measured to yield the partial pressure of carbon dioxide, leading to quantifying the absolute amount of carbon dioxide present. Colorimetric and fluoride ion selective electrode methods are commonly used to measure fluoride levels. A common colorimetric method uses sulfanilic acid azochromotrope, 1,8-Dihydroxy-2-(4-sulfophenylazo)naphthalene-3,6-disulfonic acid trisodium salt, 2-(4-Sulfophenylazo)-1,8-dihydroxy-3,6-naphthalene disulfonic acid trisodium salt, 2-(4-Sulfophenylazo)chromotropic acid trisodium salt, or sodium 1-(parasulphophenylazo)-1,8-dihydroxy-3, 6-naphthalene disulfate. These colorimetric methods are based upon the reaction between fluoride and a dark red zirconium dye to form a colorless complex anion. These methods result in a bleaching of the red color in an amount proportional to the fluoride concentration. For example, higher fluoride concentrations react with more of the zirconium-dye complex and the solution turns a lighter color. The resulting color from the colorimetric reaction may be determined photometrically, for example, using a spectrophotometer. The amount of fluoride may be determined by comparison to a similarly prepared blank vial. The absorbance of the sample reacted vial must be compared to the absorbance of the unreacted blank vial to determine the fluoride concentration of the sample reacted vial. Similar techniques, specifically the use of bleaching chemistries and comparison against blanks, are used for measuring other analytes (e.g., carbonate, mercury, borate, etc.).

However, the current analyte testing methods have limitations which are overcome by the methods and techniques as described in more detail herein. One limitation of the current techniques is that they use a bleaching chemistry not favorable to some uses and measurement systems. Additionally, the traditional colorimetric methods require the preparation of a separate "blank" vial. The extra step of preparing a blank vial can introduce error to the measurement based upon individual human techniques in preparing the blank. Also, since the traditional colorimetric technique involves the bleaching of a dye, the time for preparation and time a measurement is taken, can introduce variability in the sample reading. Additionally, because the techniques include bleaching of a dye, difficulty may arise because there may not be the same volume of starting colorimetric dye in both the blank and sample vial, thereby introducing error into the determination of the amount of analyte found in the sample. This error may result in a false positive or false negative result.

The ion selective electrode may also have disadvantages. The ion selective electrode requires the addition of an ionic strength adjustment buffer. Measurements may be affected by the omission or addition of this buffer and it requires an additional preparation step. Also, the equilibrium time for low parts per billion (ppb) levels of fluoride and other analytes is long and sensitive to both sample movement and temperature, which can in turn, lead to inaccurate results.

Accordingly, the currently claimed method includes generation of a metal organic framework (MOF). The MOF is generated in a reactive solution and contains at least one organic linker and at least one metal. The MOF is impregnated with a photoreactive species. The photoreactive species may be a dye, a chromophore, a fluorochrome, or the like. There may be multiple photoreactive species used to impregnate the MOF. The impregnated MOF is introduced into an aqueous sample. An analyte component concentration of an aqueous sample is measured. The analyte component is either fluoride, or borate, or carbonate, or mercury.

Additionally, a linker molecule (including more than one linker molecule) is selected based upon the analyte to be measured. The analyte is either fluoride, or carbonate, or borate, or mercury. Thus, the linker molecule that is selected is specific to the analyte that is being measured. The impregnated MOF contains at least one organic linker and at least one metal. The metal is selected based upon an interaction with a linker and/or the analyte to be measured. The linker is selected such that the linker is released into a solution in the presence of an analyte. The dissociation of the MOF and release of a photoreactive species allows the dye to come into contact with the analyte in the aqueous sample. In an embodiment, the dye is a colorimetric dye, a fluorescent dye, the like, or a combination of dyes.

Some current fluoride or other analyte tests use a bleaching chemistry. In other words, the color dissipates over time in the presence of fluoride or another analyte, which may or may not become colorless. The impregnated MOF disclosed herein starts with a solution that does not exhibit a significant absorbance or fluorescence at the desired measurement wavelength. The absorbance or fluorescence at the desired measurement wavelength, or over a certain wavelength range, increases when fluoride is present in solution. Thus, the impregnated MOF discussed herein does not require the measurement of a "loss of color" or "loss of fluorescence" as in the bleaching chemistries and also does not require the preparation of a "blank", thereby providing a system that provides more accurate measurements of the analyte than conventional systems.

A method in which the solution turns from colorless to exhibiting a color, or non-emitting to exhibiting fluorescence, has advantages. Accordingly, the method is able to get an initial measurement because the measurement is colorless or does not exhibit a significant absorbance or fluorescence at the desired measurement wavelength, thereby eliminating the need for a "blank". During the reaction, the sample turns a color or emits fluorescent light which can then be measured. In other words, an embodiment goes from colorless to some degree of color, for example, colorless to pink to red over time, thereby allowing for more accurate measurements than attempting to measure a colored sample going to a less colored sample as in the bleaching chemistries. In another embodiment, a sample that does not fluoresce may begin to emit blue fluorescent light upon excitation with a lower wavelength of light, thereby allowing for more accurate measurements than attempting to measure a brightly fluorescent sample going to a less fluorescent sample as in the bleaching chemistries. Further embodiments include the possibility of absorbing or emitting other colors such as yellow, green, or any other color measurable by laboratory apparatus. The absorbance can be translated to the concentration of fluoride, carbonate, or other target analyte in solution. In an embodiment, this relationship is 1:1 between the absorbance and a type of analyte to be measured, however the absorbance or fluorescence may be different for other photoreactive species. Therefore, the techniques and methods as described herein may be used with current technologies.

The illustrated example embodiments will be best understood by reference to the figures. The following description is intended only by way of example, and simply illustrates certain example embodiments.

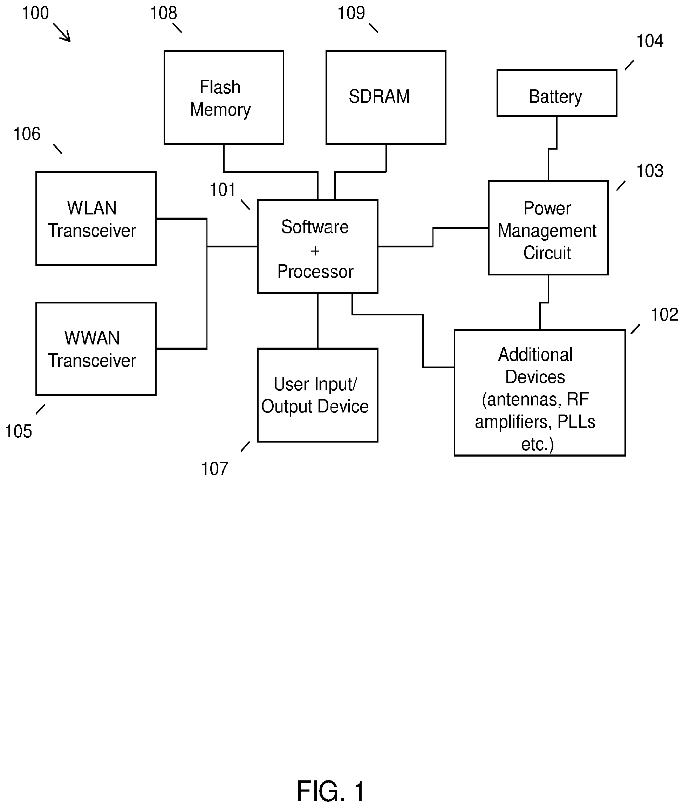

While various other circuits, circuitry or components may be utilized in information handling devices, with regard to an instrument for analyte measurement according to any one of the various embodiments described herein, an example is illustrated in FIG. 1. For example, the device circuitry as described in FIG. 1 may be used for communicating measurements to another device or may be used as the device for receiving measurements. Device circuitry 100 may include a measurement system on a chip design found, for example, a particular computing platform (e.g., mobile computing, desktop computing, etc.) Software and processor(s) are combined in a single chip 101. Processors comprise internal arithmetic units, registers, cache memory, busses, I/O ports, etc., as is well known in the art. Internal busses and the like depend on different vendors, but essentially all the peripheral devices (102) may attach to a single chip 101. The circuitry 100 combines the processor, memory control, and I/O controller hub all into a single chip 101. Also, systems 100 of this type do not typically use SATA or PCI or LPC. Common interfaces, for example, include SDIO and I2C.

There are power management chip(s) 103, e.g., a battery management unit, BMU, which manage power as supplied, for example, via a rechargeable battery 104, which may be recharged by a connection to a power source (not shown). In at least one design, a single chip, such as 101, is used to supply BIOS like functionality and DRAM memory.

System 100 typically includes one or more of a WWAN transceiver 105 and a WLAN transceiver 106 for connecting to various networks, such as telecommunications networks and wireless Internet devices, e.g., access points. Additionally, devices 102 are commonly included, e.g., a transmit and receive antenna, oscillators, RF amplifiers, PLLs, etc. System 100 includes input/output devices 107 for data input and display/rendering (e.g., a computing location located away from the single beam system that is easily accessible by a user). System 100 also typically includes various memory devices, for example flash memory 108 and SDRAM 109.

It can be appreciated from the foregoing that electronic components of one or more systems or devices may include, but are not limited to, at least one processing unit, a memory, and a communication bus or communication means that couples various components including the memory to the processing unit(s). A system or device may include or have access to a variety of device readable media. System memory may include device readable storage media in the form of volatile and/or nonvolatile memory such as read only memory (ROM) and/or random access memory (RAM). By way of example, and not limitation, system memory may also include an operating system, application programs, other program modules, and program data. The disclosed system may be used in an embodiment to perform analyte measurement of an aqueous sample.

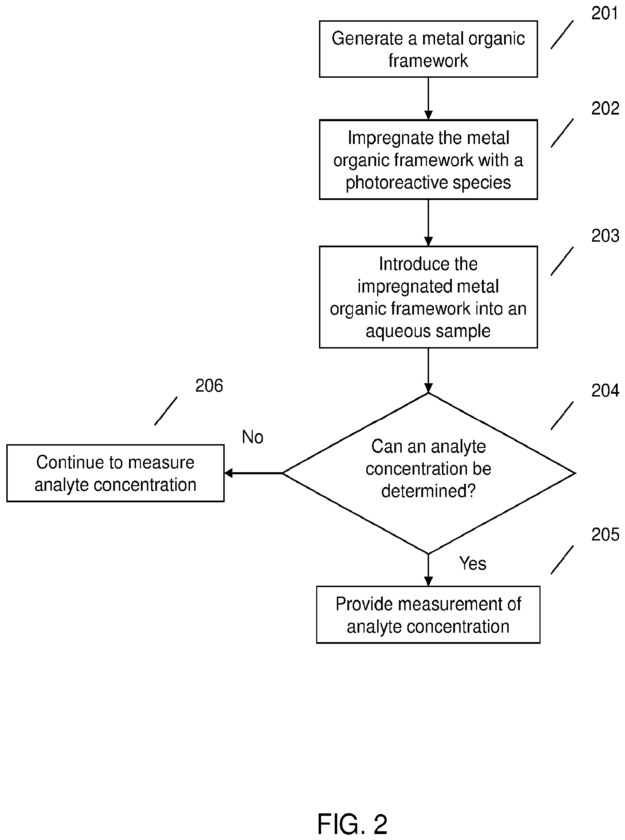

Referring now to FIG. 2, a method provides a measurement of an analyte concentration in an aqueous environment. The analyte is either fluoride, or borate, or carbonate, or mercury. For ease of reading, fluoride and/or carbonate may be used as an exemplar analyte, however other analytes are disclosed and contemplated. A MOF structure is capable of detecting different analytes. The analyte enters the pore of a MOF structure. The analyte binds the metal of a MOF thus displacing the linker and releasing the linker into solution. The linker is a photoreactive species such as a chromophore or a fluorochrome. In an embodiment, the MOF contains more than one type of linker molecule. The MOF is impregnated with a photoreactive species. A photoreactive species is selected based upon an analyte to be detected and/or measured. In other words, one photoreactive species is used when measuring fluoride, while another photoreactive species is selected when measuring carbonate.

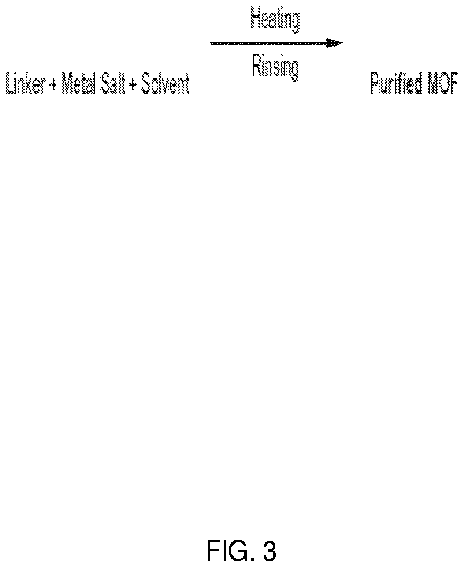

At 201, a metal organic framework (MOF) is generated. The synthesis of the MOF itself is performed by standard techniques. The MOF is added in excess to the preparation technique. An exemplar method of making a purified MOF is illustrated in FIG. 3. A MOF contains at least one metal and at least one organic linker. Different combinations of metals and linkers yield a wide range of properties for analyte detection. In an embodiment, the at least one metal and the at least one organic linker are used in different combinations. For example, two different metals are used, or two or more different linkers are used. Thus, different metals and/or linkers may be selected based upon the analyte to be detected and/or measured.

Aluminum or zirconium metal are used in the generation of the MOF. In an embodiment, these metals are dissolved by fluoride or carbonate. The metal is a metal salt. The metal salt has as the cation aluminum (Al³⁺) or zirconium (Zr⁴⁺), Selection of the metal is determined based upon the analyte to be measured. In an embodiment, the linker is selected based upon the photosensitive species to be used. Alternatively, the linkers are selected based upon their own signal created by an analyte presence. The linker is terephthalic acid, 2-aminoterephthalic acid, 2-nitroterephthalic acid, 4,4'-biphenyldicarboxylic acid, 1,4-naphthalenedicarboxylic acid or trimesic acid.

At 202, the MOF is impregnated with a dye or other photoreactive species. For ease of reading, the example of a dye is described herein. A MOF is a microporous material and soaked in a dye solution. This soaking of the MOF in a dye is referred to as impregnating the MOF. The resulting dye and MOF product are referred to as an impregnated MOF or a dye-impregnated MOF. The MOF is impregnated by soaking in a dye solution overnight. Other soak times may be used to achieve proper soaking of a dye. In an embodiment, two hours are sufficient for the impregnating step. The soaking solution may use a mechanical means such as stirring for proper impregnation of the dye into the MOF. In an embodiment, ethanol solution is used during the soaking. After soaking, there is a washing or rinsing step. The washing removes excess dye. The washing ensures that any dye present in the impregnated MOF is within the matrix or pores of the MOF. The impregnated MOF is filtered. The impregnated MOF is centrifuged for separation of excess dye. At least one cycle of washing and centrifugation are performed. The MOF deteriorates in the presence of an analyte, and, therefore, releases the dye into an aqueous solution.

The dye is selected based upon metal linker and dye interaction. For example, a linker that is stable with a dye is used to maintain the dye within the MOF until an analyte to be measured is present. The absorbance of the dye is proportional to the analyte to be measured. The measured absorbance is dependent on the amount of dye in a MOF matrix or micropores. A MOF is selected based upon a metal organic framework pore size as compared to a dye size. Accordingly, a MOF is selected such that a dye fits inside the matrix or pores of the MOF. In an embodiment, a dye is not chemically immobilized.

At 203, an analyte is measured using an impregnated MOF. The analyte (i.e., fluoride, mercury, borate, carbonate) is an ion in an aqueous sample. When the MOF is introduced to the aqueous sample, the analyte binds to the metal and removes the metal from the framework to release the linkers and the dye into solution. The concentration of linker is then directly measured. The free linker interacts with the analyte in the aqueous solution, thereby providing a colorimetric and/or fluorescent change to the aqueous sample. Thus, the colorimetric and/or fluorescent change is detected and measured by appropriate measurement devices.

The impregnated MOF is introduced into a chamber, vessel, or the like containing an aqueous sample for analyte measurement. Alternatively, the aqueous sample is introduced to the impregnated MOF. The combination of the aqueous sample and the impregnated MOF may occur in the same or in a different chamber in which an absorption measurement occurs. Pumps, valves, and piping control and direct the flow of reagents. These systems are automated or controlled by a processor.

At 203, absorbance of a linker is measured. Absorbance measurement of a linker may be independent or in conjunction with absorbance measurement of a dye. For example, an absorbance scan in the range of 250 - 800 nm measures two distinct peaks or signal. For example, a linker may show up at 300 nm and a dye at 500 nm. In an embodiment, both the absorbance value of the linker and the dye are measured. The absorbance of the linker is proportional to the analyte to be measured. Colorimetric or fluorescent methods may be used to measure a linker's absorbance or fluorescence. A fluorescent method is more sensitive.

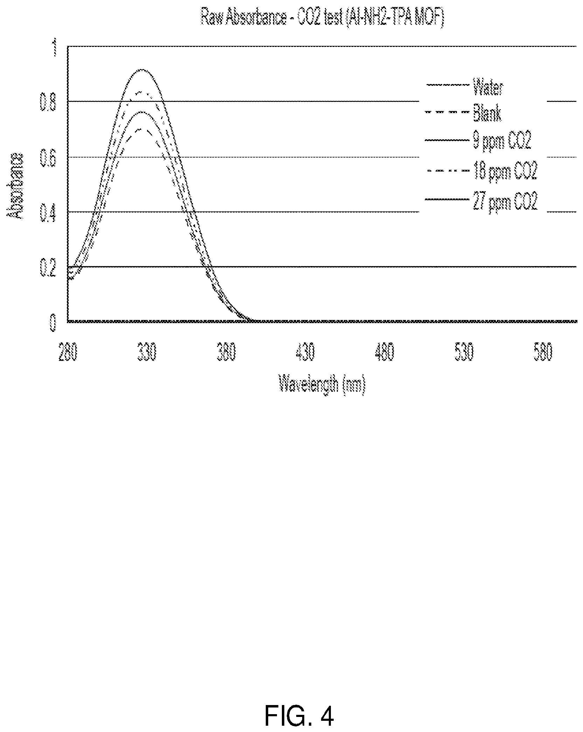

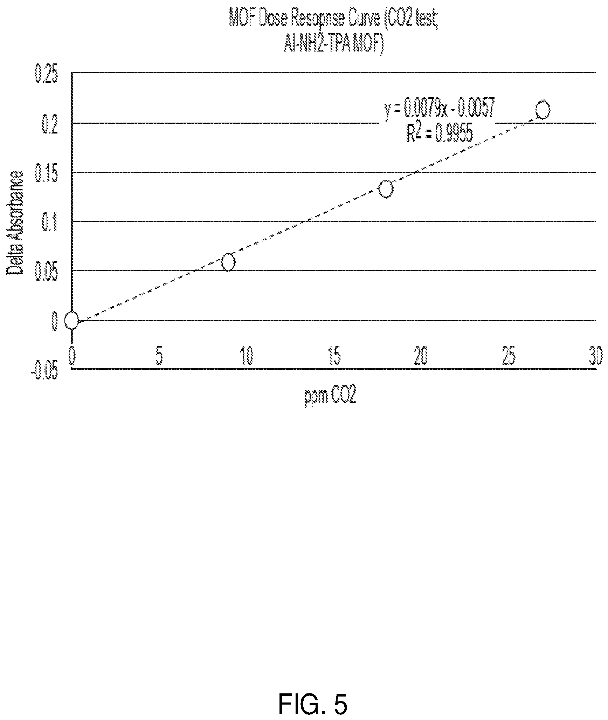

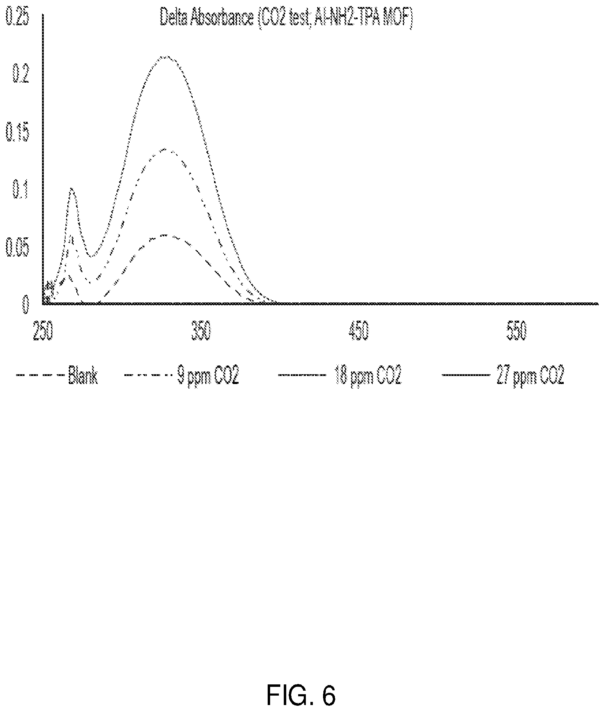

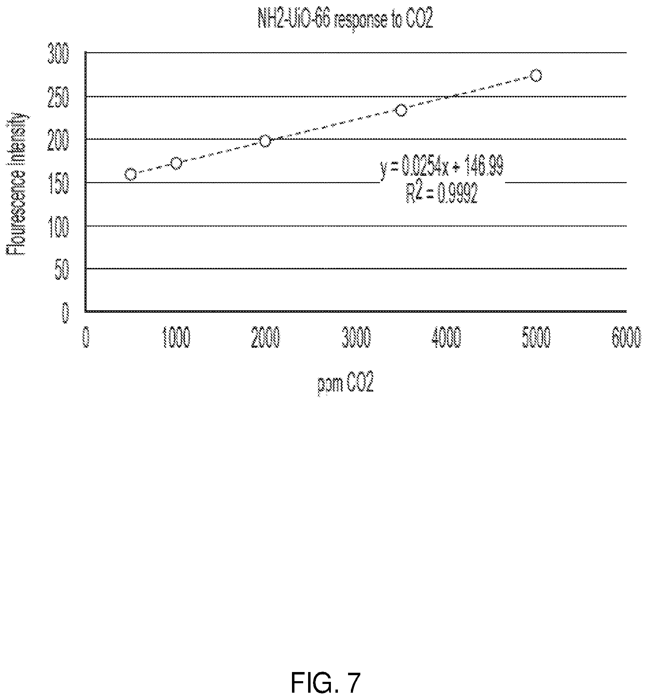

At 204 an analyte concentration is determined. The presence of an analyte in an aqueous solution causes a shift in the absorption of the linker and the dye. Examples of this shift in absorbance and dose response curves for various dyes, MOFs, and conditions are illustrated in Figures attached herewith in the Appendix. FIGs. 4 - 6 illustrate examples of raw absorbance, MOF dose response curve, and delta absorbance of a CO₂ test of Al-NH₂-TPA MOF. FIG. 7 illustrates an example CO₂ fluorescence intensity response curve. Therefore, the absorbance or fluorescence at a specified wavelength, of an aqueous sample containing an analyte is correlated to the concentration of the analyte in the aqueous solution. The absorbance or fluorescence wavelength is in the visible light spectrum, ultraviolet spectrum, or the like. This change in absorbance or fluorescence is measurable and able to be correlated to the concentration of analyte within the aqueous solution. Absorption or fluorescence curves are generated for a range of analyte concentrations, for different dyes, for any different condition that may affect absorption or fluorescence values (e.g., temperature, sample content, turbidity, viscosity, measurement apparatus, aqueous sample chamber, etc.), or the like.

At 205, a measurement of analyte concentration is provided. The change in absorption is measured using a spectrophotometer. Spectrophotometry is a measurement of reflection or transmission properties of a sample measured at a wavelength. Spectrophotometry is a quantitative measure of how much light is absorbed by a material. Additionally or alternatively, the change in is measured using a spectrofluorometer. Spectrofluorometry is measurement of emission and/or transmission properties of a sample measured at a wavelength. Spectrofluorometry is a quantitative measure of how much light is emitted by a material after absorbing light. This material may be linkers (according to the present invention) or photoreactive species released from an impregnated MOF sensitive to fluoride in an aqueous sample. The absorbance or fluorescence wavelength may be in a visible or ultraviolet wavelength, as chromophores, fluorochromes, or the like are used. The change in absorption or fluorescence may also be measured using other colorimetric or fluorometric measurement devices.

Alternatively or additionally, fluoride and/or carbonate concentration measurement may be at periodic intervals set by the user or preprogrammed frequencies in the device. Measurement of fluoride and/or carbonate by a device allows for real time data with very little human involvement in the measurement process. Cleaning of the colorimetric and/or fluorometric chamber is required at an unspecified time interval. A programmed calibration curve is entered into the device.

A chamber, vessel, cell, chamber, or the like contain an aqueous sample, at least one MOF that is impregnated with photoreactive species, and associated reagents. A device contains one or more bottles of reagents which contain necessary reagents such as at least one impregnated MOF, buffers, or any reagent that is not premixed before the measuring process. The reagents contained in the one or more bottles may be pump fed or gravity fed. The flow of the reagents is metered to ensure proper volume delivery to the measurement cell. The aqueous sample may be fed through a pressured inlet, a vessel, or the like. The aqueous sample is introduced into the measurement chamber by a pump or gravity fed. The sampling device may be in series or parallel to an aqueous flow. The device has a system to ensure proper mixing of the aqueous sample, impregnated MOF, and related reagents.

The analyte or fluoride measurement may be an output upon a device in the form of a display, printing, storage, audio, haptic feedback, or the like. Alternatively or additionally, the output may be sent to another device through wired, wireless, fiber optic, Bluetooth^{®}, near field communication, or the like. An embodiment may use an alarm to warn of an analyte measurement or concentration outside acceptable levels. An embodiment may use a system to shut down water output or shunt water from sources with unacceptable levels of an analyte. For example, an analyte measuring device may use a relay coupled to an electrically actuated valve, or the like.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium such as a non-signal storage device, where the instructions are executed by a processor. In the context of this document, a storage device is not a signal and "non-transitory" includes all media except signal media.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and products according to various example embodiments. It will be understood that the actions and functionality may be implemented at least in part by program instructions. These program instructions may be provided to a processor of a device, e.g., a hand-held measurement device such as illustrated in FIG. 1, or other programmable data processing device to produce a machine, such that the instructions, which execute via a processor of the device, implement the functions/acts specified.

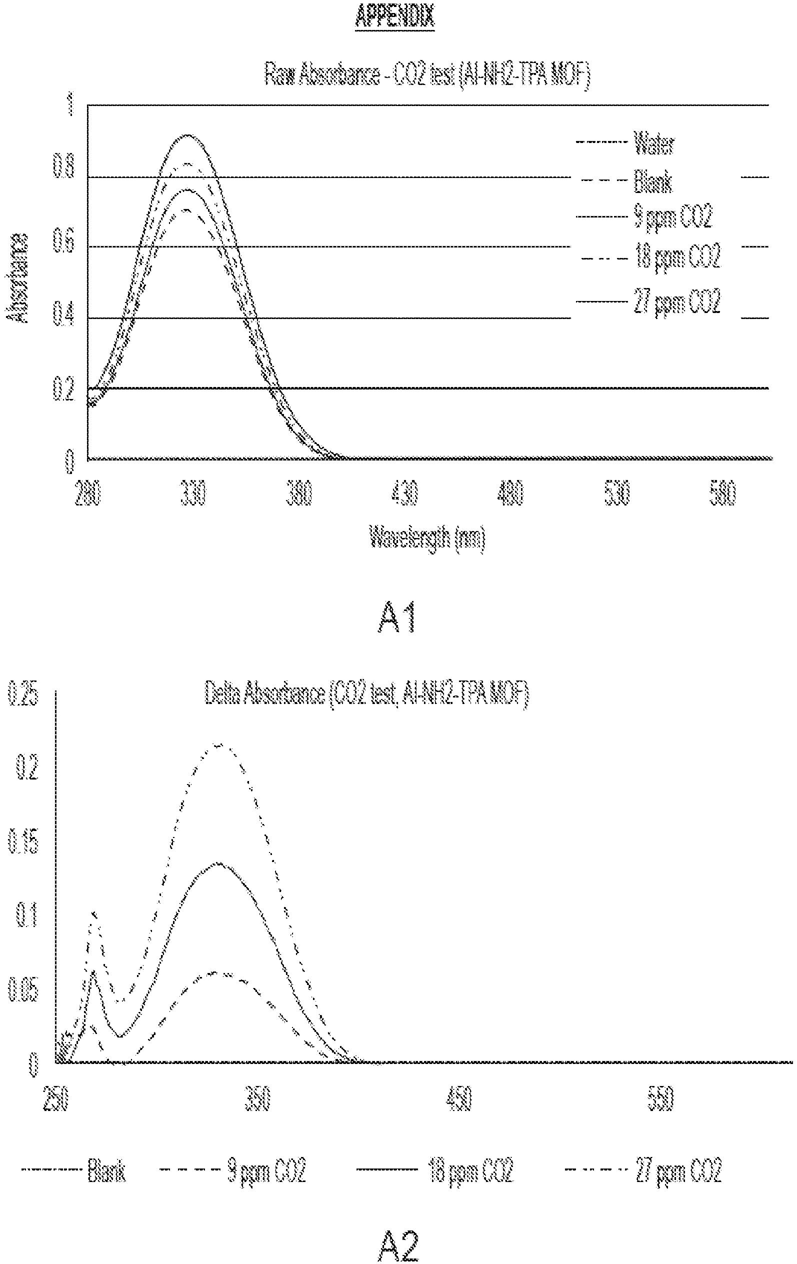

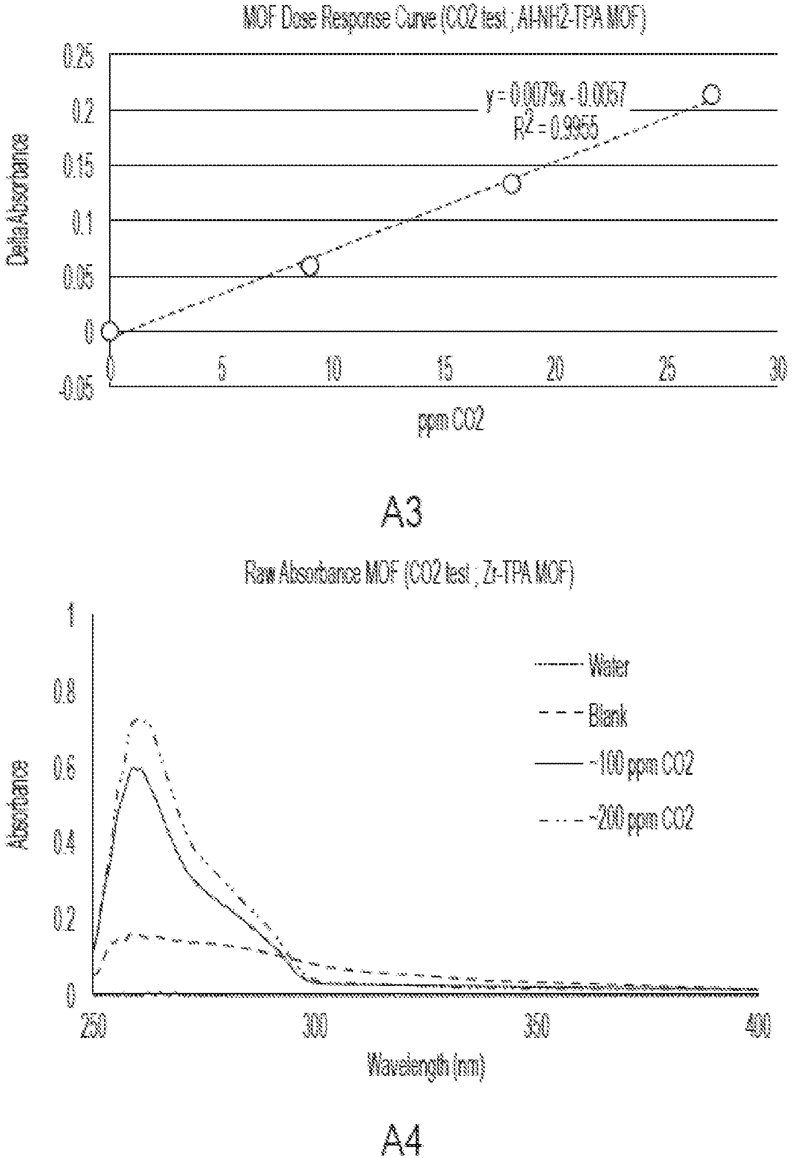

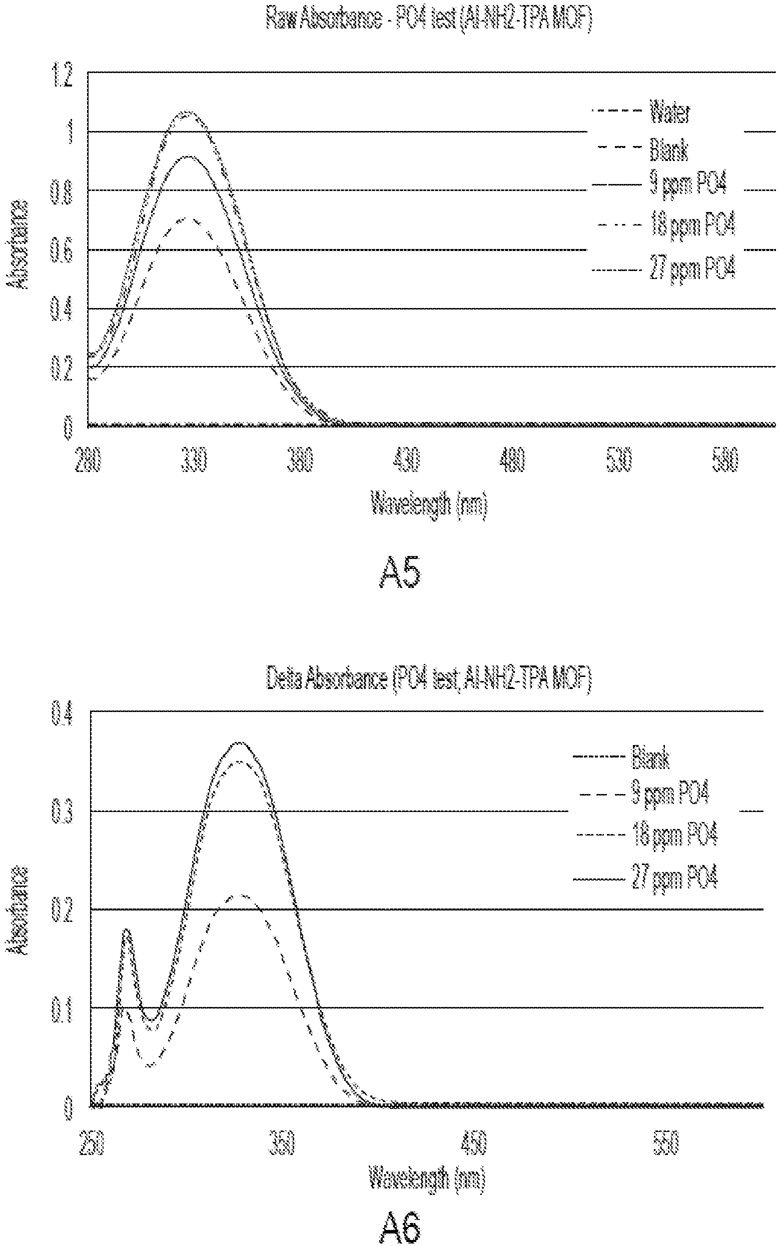

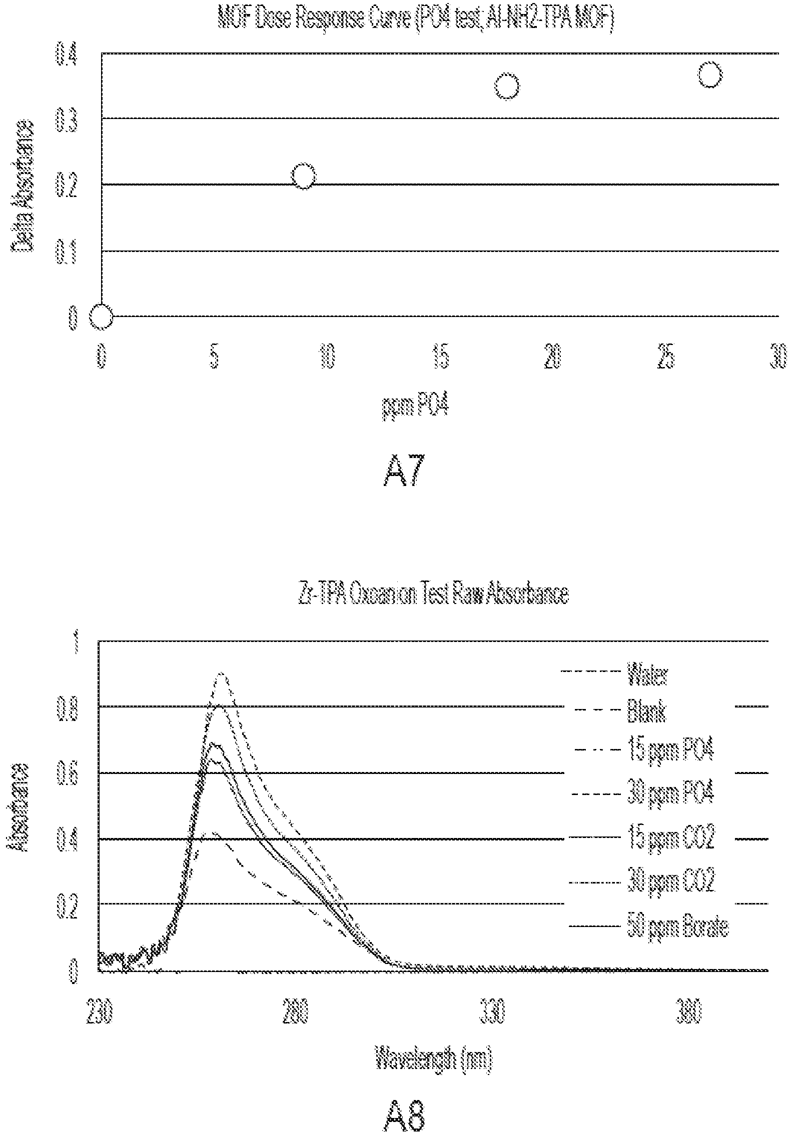

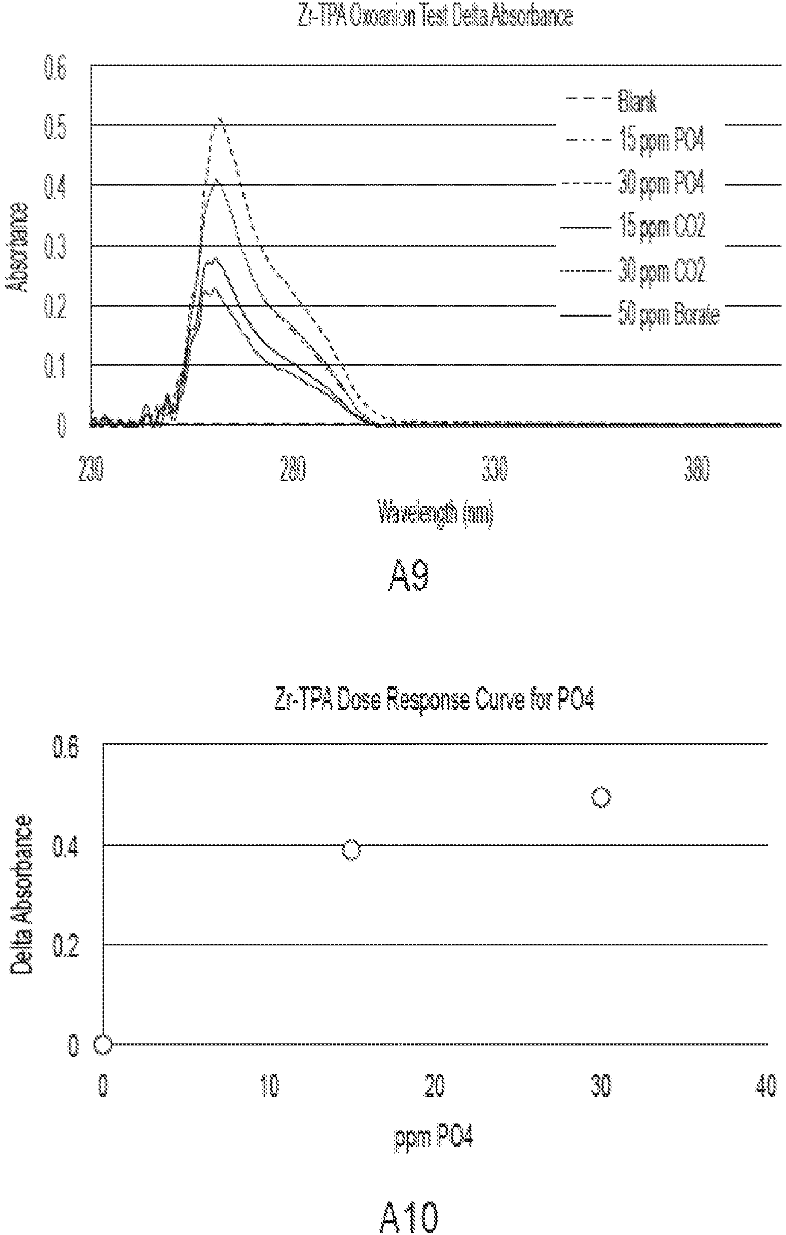

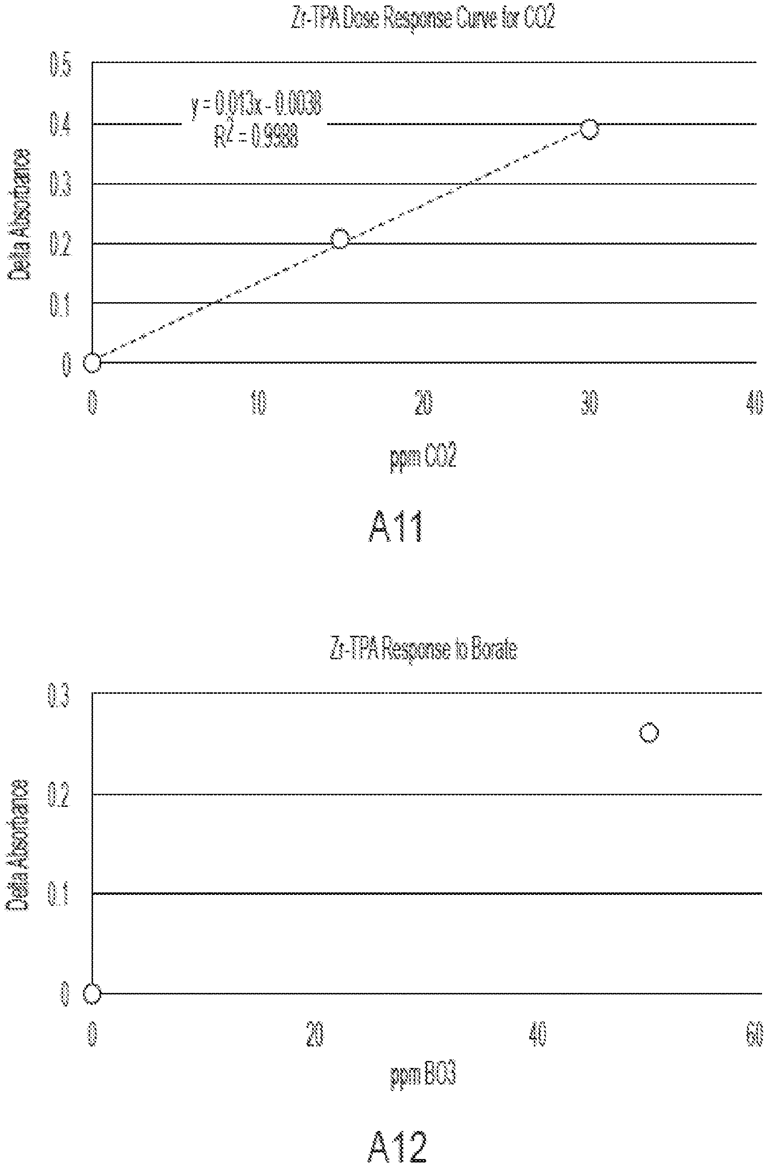

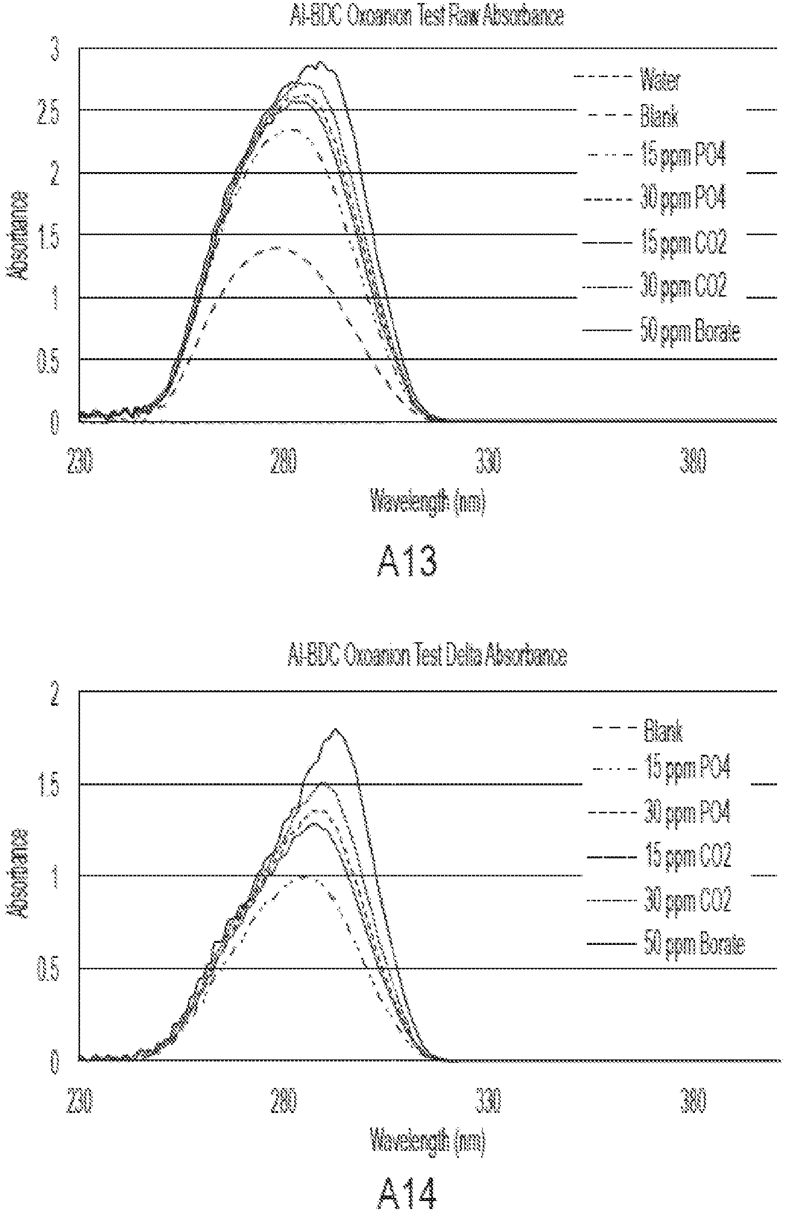

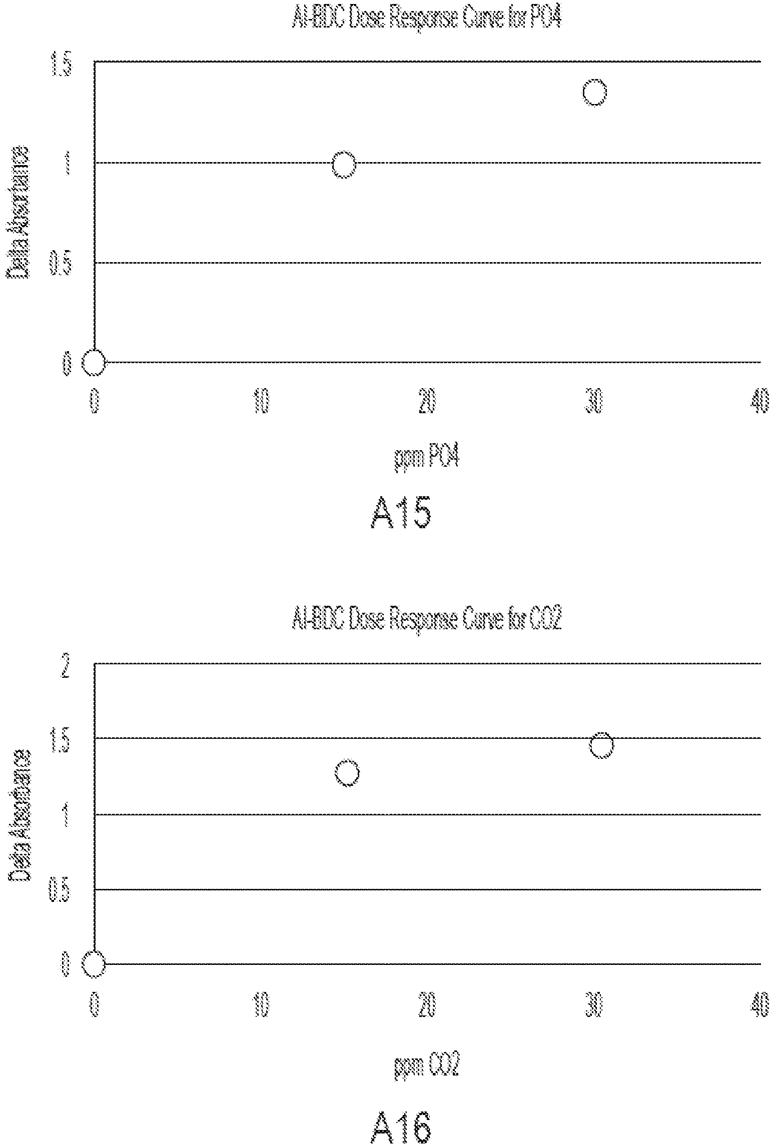

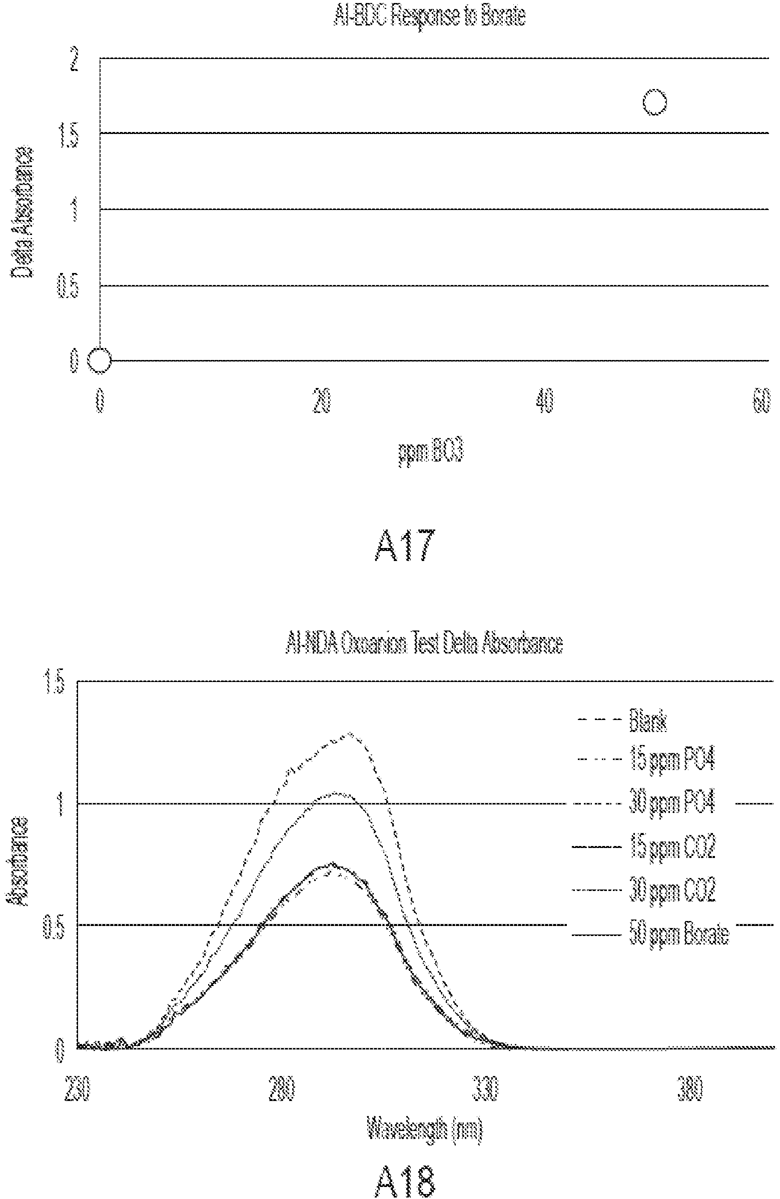

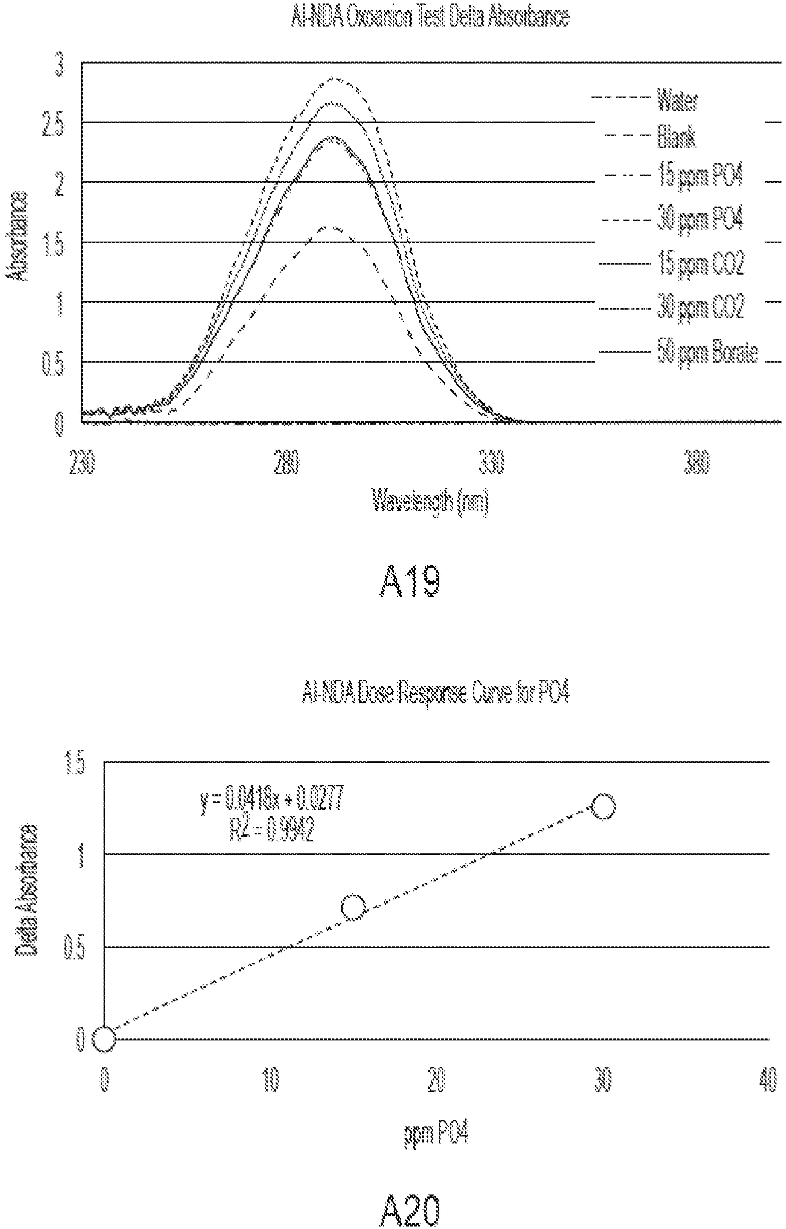

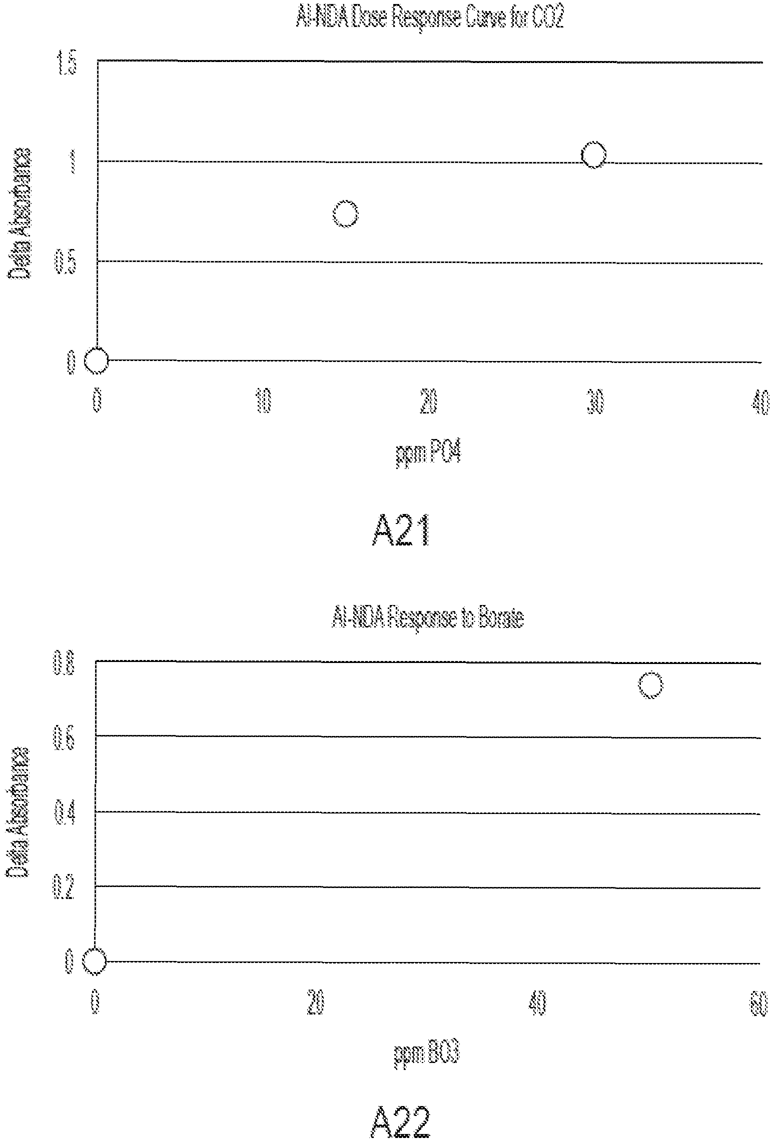

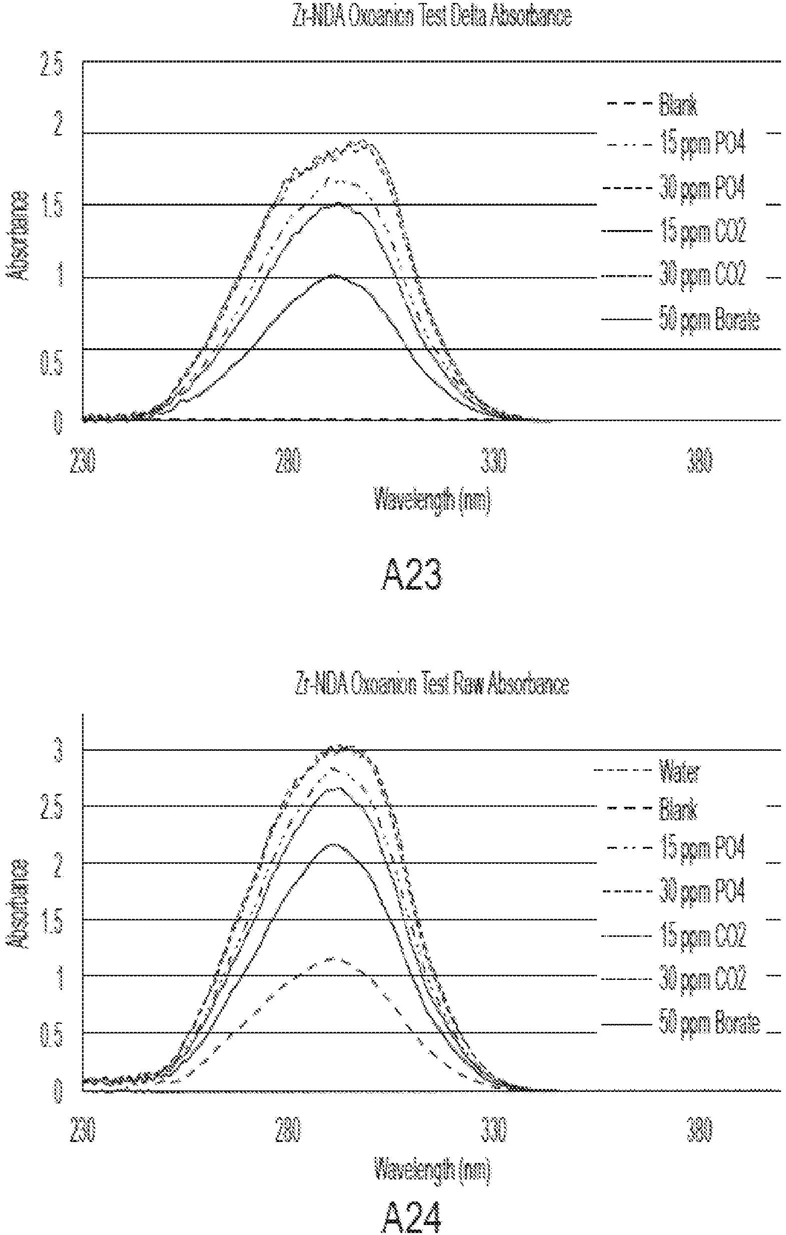

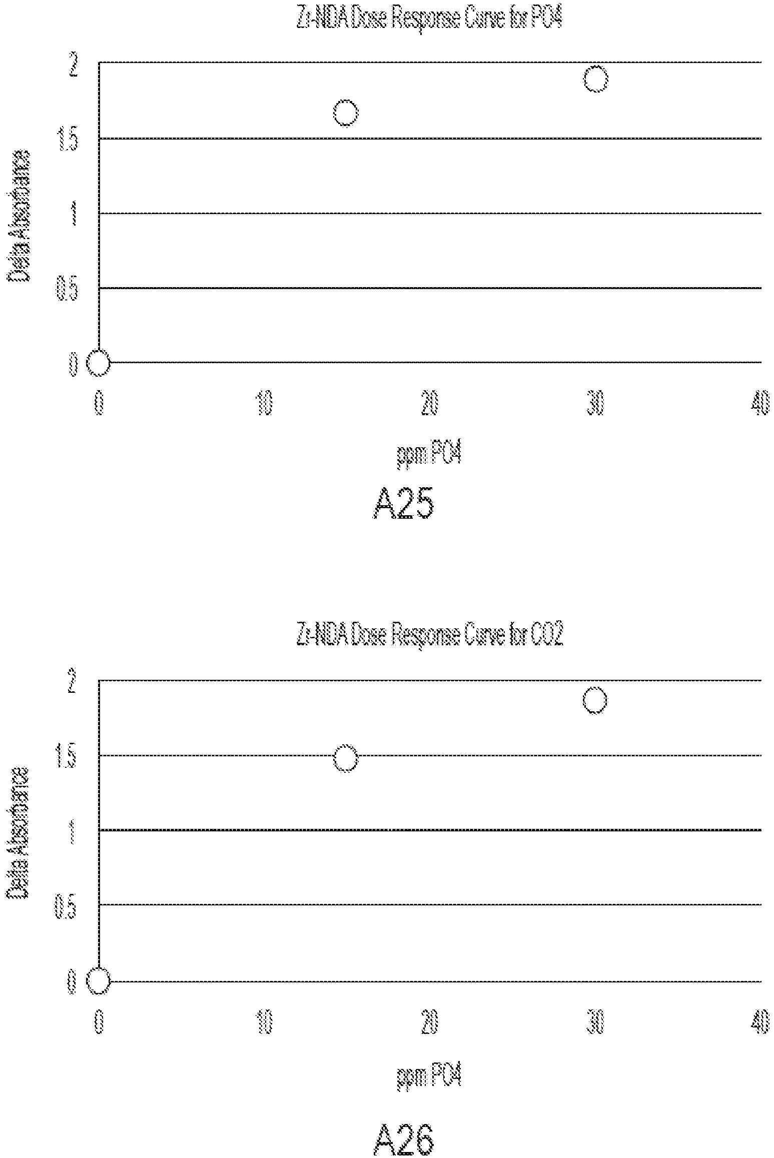

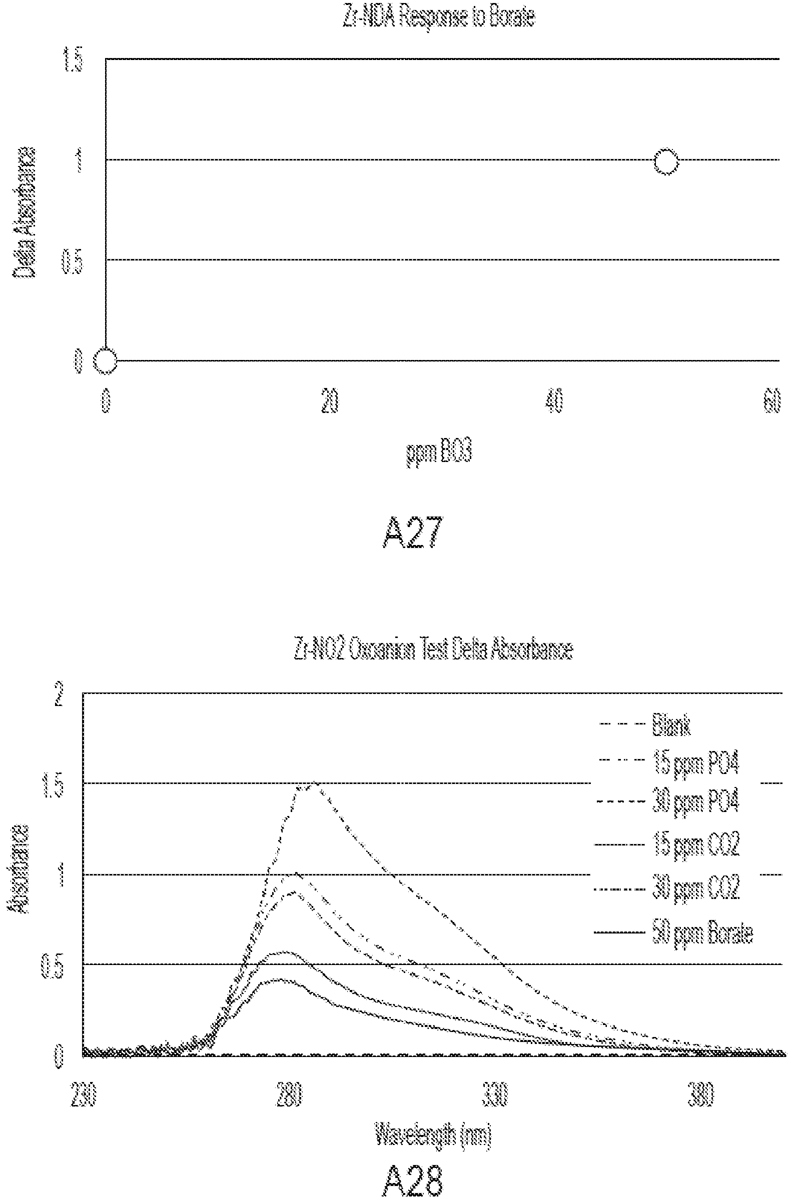

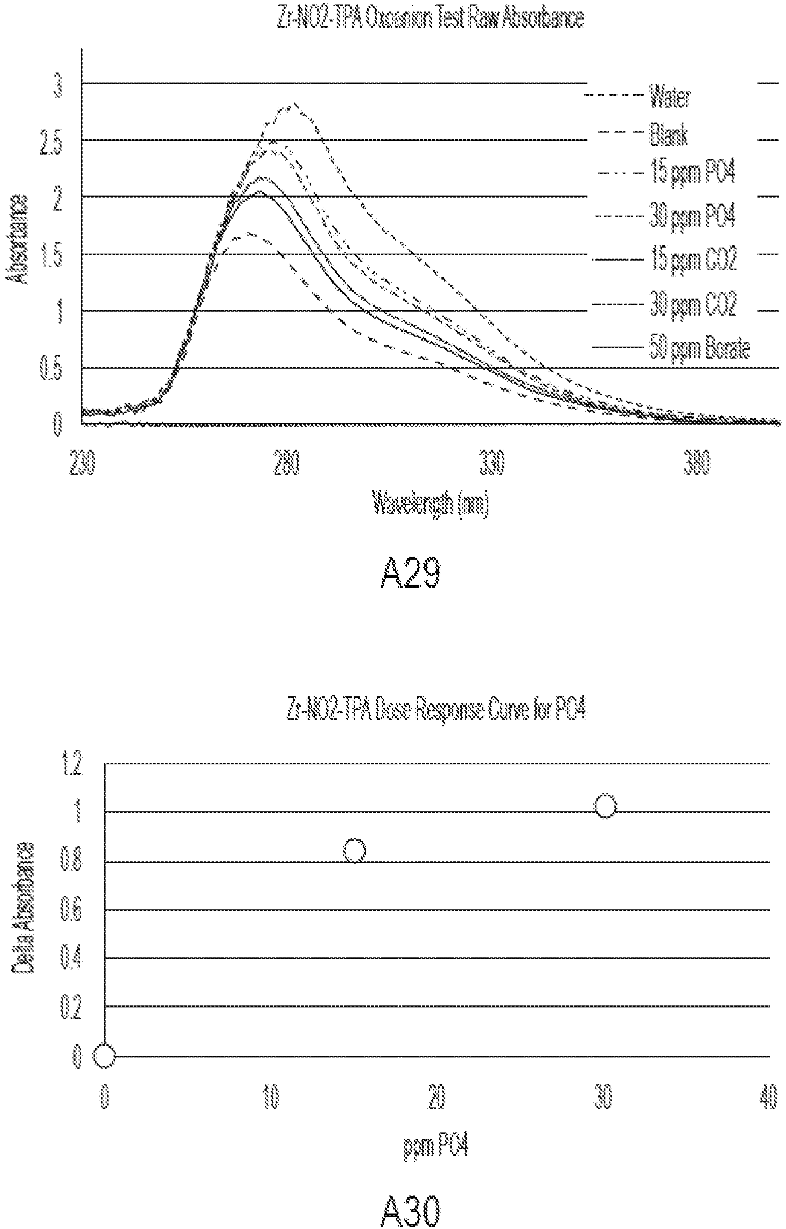

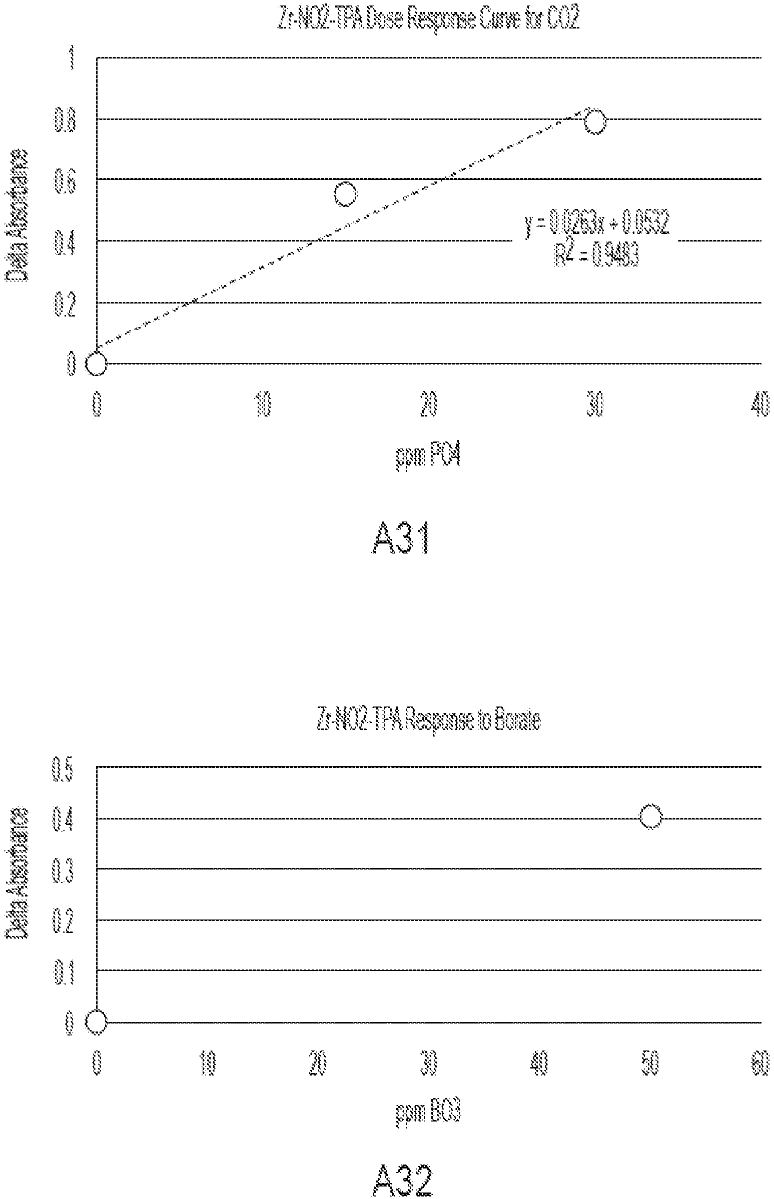

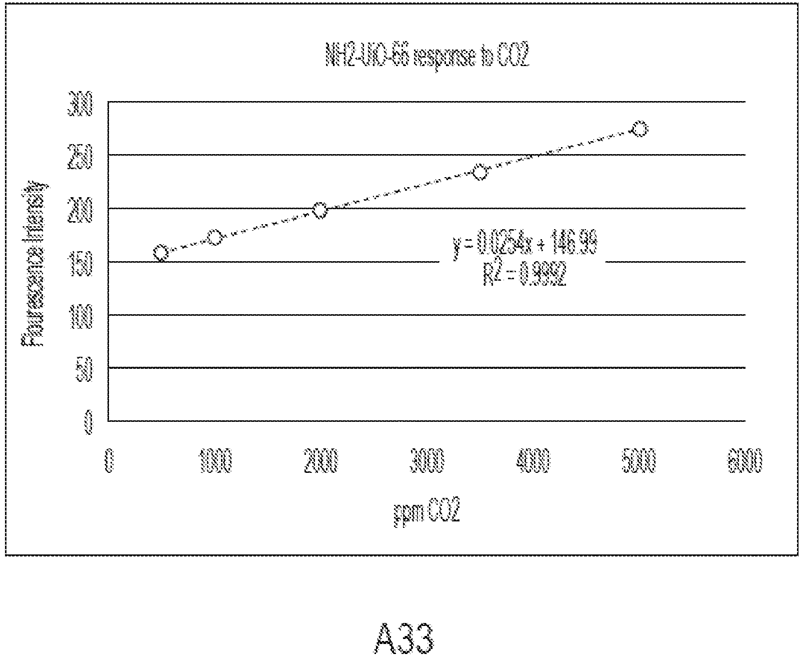

## Claims

1. A method for measuring an analyte ion component of an aqueous sample using a photoreactive species, comprising:
generating (201) a metal organic framework by combining, in a reactive solution, at least one organic linker and at least one metal salt;
wherein the analyte ion component is selected from the group consisting of: fluoride, carbonate, borate, and mercury;
wherein the at least one linker is selected from the group consisting of: terephthalic acid, 2-aminoterephthalic acid, 2-nitroterephthalic acid, 4,4'-biphenyldicarboxylic acid, 1,4-naphthalenedicarboxylic acid, and trimesic acid; and
wherein the at least one metal salt is selected from the group consisting of: aluminum nitrate nonahydrate, aluminum chloride hexahydrate, zirconyl chloride, and zirconium (IV) tetrachloride;
impregnating (202) the metal organic framework with the photoreactive species;
introducing (203) the impregnated metal organic framework into an aqueous sample, whereby the analyte ion component binds to the at least one metal salt and removes the metal from the metal organic framework to release the at least one linker and the photoreactive species into solution;
measuring (204) an analyte ion component concentration of the aqueous sample, wherein the measuring comprises measuring a change in the aqueous sample, wherein the change is responsive to the analyte ion component dissolving the at least one metal salt, and releasing the at least one organic linker, thereby providing a colorimetric and/or fluorescent change to the aqueous sample; and
wherein the measuring comprises measuring an absorbance or fluorescence at a given wavelength of the at least one linker.

2. The method of claim 1, wherein the photoreactive species is a chromophore.

3. The method of claim 1, wherein the photoreactive species is a fluorochrome.

## Patentansprüche

1. Verfahren zum Messen einer Analytenionenkomponente einer wässrigen Probe unter Verwendung einer photoreaktiven Spezies, umfassend:
Erzeugen (201) eines metallorganischen Gerüsts durch Kombinieren mindestens eines organischen Verbinders und mindestens eines Metallsalzes in einer reaktiven Lösung;
wobei die Analytenionenkomponente aus der Gruppe ausgewählt ist, die aus Fluorid, Carbonat, Borat und Quecksilber besteht;
wobei der mindestens eine Verbinder aus der Gruppe ausgewählt ist, die besteht aus: Terephthalsäure, 2-Aminoterephthalsäure, 2-Nitroterephthalsäure, 4,4'-Biphenyldicarbonsäure, 1,4-Naphthalindicarbonsäure und Trimesinsäure ausgewählt ist; und wobei das mindestens eine Metallsalz aus der Gruppe ausgewählt ist, die aus Aluminiumnitrat-Nonahydrat, Aluminiumchlorid-Hexahydrat, Zirkonylchlorid und Zirkonium(IV)-tetrachlorid besteht;
Imprägnieren (202) des metallorganischen Gerüsts mit der photoreaktiven Spezies;
Einbringen (203) des imprägnierten metallorganischen Gerüsts in eine wässrige Probe, wodurch die Analyt-Ionenkomponente an das mindestens eine Metallsalz bindet und
das Metall aus dem metallorganischen Gerüst entfernt, um den mindestens einen Verbinder und die photoreaktive Spezies in Lösung freizusetzen;
Messen (204) einer Analyt-Ionenkomponentenkonzentration der wässrigen Probe, wobei das Messen das Messen einer Veränderung in der wässrigen Probe umfasst,
wobei die Veränderung darauf reagiert, dass die Analyt-Ionenkomponente das mindestens eine Metallsalz auflöst und den mindestens einen organischen Linker freisetzt, wodurch eine kolorimetrische und/oder fluoreszierende Veränderung der wässrigen Probe hervorgerufen wird; und
wobei das Messen das Messen einer Absorption oder Fluoreszenz bei einer gegebenen Wellenlänge des mindestens einen Verbinder umfasst.

2. Verfahren nach Anspruch 1, wobei die photoreaktive Spezies ein Chromophor ist.

3. Verfahren nach Anspruch 1, wobei die photoreaktive Spezies ein Fluorochrom ist.

## Revendications

1. Procédé de mesure d'un composant ionique analyte d'un échantillon aqueux utilisant une espèce photoréactive, comprenant :
la génération (201) d'une structure organométallique en combinant, dans une solution réactive, au moins un lieur organique et au moins un sel métallique ;
dans lequel le composant ionique analyte est choisi dans le groupe constitué par : le fluorure, le carbonate, le borate et le mercure ;
dans lequel le au moins un lieur est choisi dans le groupe constitué par : l'acide téréphtalique, l'acide 2-aminotéréphtalique, l'acide 2-nitrotéréphtalique, l'acide 4,4'-biphényldicarboxylique, l'acide 1,4-naphtalènedicarboxylique, l'acide trimésique ; et
dans lequel le au moins un sel métallique est choisi dans le groupe constitué par : le nitrate d'aluminium nonahydraté, le chlorure d'aluminium hexahydraté, le chlorure de zirconyle et le tétrachlorure de zirconium (IV) ;
l'imprégnation (202) de la structure organométallique avec l'espèce photoréactive ;
l'introduction (203) de la structure organométallique imprégnée dans un échantillon aqueux, moyennant quoi le composant ionique analyte se lie au au moins un sel métallique et extrait le métal de la structure organométallique pour libérer le au moins un lieur et l'espèce photoréactive en solution ;
la mesure (204) d'une concentration du composant ionique analyte de l'échantillon aqueux, dans lequel la mesure comprend la mesure d'un changement dans l'échantillon aqueux, dans lequel le changement est sensible à la dissolution du au moins un sel métallique par le composant ionique analyte, et à la libération du au moins un lieur organique, fournissant ainsi un changement colorimétrique et/ou fluorescent à l'échantillon aqueux ; et
dans lequel la mesure comprend la mesure d'une absorbance ou d'une fluorescence à une longueur d'onde donnée dudit au moins un lieur.

2. Procédé selon la revendication 1, dans lequel l'espèce photoréactive est un chromophore.

3. Procédé selon la revendication 1, dans lequel l'espèce photoréactive est un fluorochrome.
